# EUROPÄISCHE PATENTSCHRIFT

(11) **EP 3 748 595 B1**
(45) Veröffentlichungstag und Bekanntmachung des Hinweises auf die Patenterteilung: **03.11.2021**
(21) Anmeldenummer: 20174473.7
(22) Anmeldetag: 13.05.2020
(51) Int. Cl.: G08B 21/02, G08B 21/04, G08B 21/22, G08B 21/24, G08B 25/01

(54) **VORRICHTUNG UND VERFAHREN ZUR ÜBERWACHUNG EINER FAHRGASTZELLE**
DEVICE AND METHOD FOR MONITORING A PASSENGER COMPARTMENT
DISPOSITIF ET PROCÉDÉ DE SURVEILLANCE D'UN HABITACLE POUR LES PASSAGERS

(30) Priorität: 23.05.2019 DE 102019113839
(43) Veröffentlichungstag der Anmeldung: 09.12.2020
(73) Patentinhaber: IndiKar Individual Karosseriebau GmbH, 08112 Wilkau-Hasslau (DE); 3dvisionlabs GmbH, 09126 Chemnitz (DE)
(72) Erfinder: Heß, Markus, 09465 Sehmatal OT Sehma (DE); Meinel, Lars, 09126 Chemnitz (DE); Findeisen, Michel, 09306 Erlau OT Milkau (DE); Gerschewski, Ronald, 08056 Zwickau (DE)
(74) Vertreter: Rumrich, Gabriele

(56) Entgegenhaltungen:
- DE-A1-102011 109 564
- US-A1- 2001 029 416
- US-A1- 2006 018 518
- US-A1- 2007 176 402
- US-A1- 2007 289 799
- US-A1- 2017 355 377

## Beschreibung

Die Erfindung betrifft eine Vorrichtung und ein Verfahren zur Überwachung einer Fahrgastzelle und deren Insassen und findet insbesondere für Sonderfahrzeuge und autonom fahrende Fahrzeuge Anwendung.

Beispielsweise aus dem Artikel "ZF Friedrichshafen AG - Presseinformation 16.10.2018 URL:http://press.zf.com/press/de/releases/release_2993.html " ist eine Vorrichtung zur Überwachung einer Fahrgastzelle eines Fahrzeuges und seiner Insassen unter Verwendung eines optischen Sensors zur Erfassung von Bildinformationen bekannt. Der Sensor ist oberhalb der Sitzposition angeordnet und erfasst die Insassen und deren Sitzposition. Nachteiligh ist, dass mit diesem Sensor nicht die komplette Fahrgastzelle überwacht wird.
Aus der Druckschrift WO 01/72557 A1 ist eine Vorrichtung und ein Verfahren zum Erfassen eines Objektes oder einer Person im Innenraum eines Fahrzeuges bekannt. Im Innenraum des Fahrzeuges ist ein Laser zum Aussenden von Laserlichtpulsen und ein optischer Empfänger oder ein Sensor zum Empfangen von, an dem Objekt oder der Person reflektierten Laserlichtpulsen vorgesehen.
Der Laser ist mit einer Erfassungseinrichtung verbunden, die fortlaufend die Empfangsleistung erfasst und bei Erreichen Eines, einer hinreichenden Empfangsleistung entsprechenden Grenzwertes den Laserlichtpuls abschaltet. Die Vorrichtung ist vorzugsweise im Dachhimmel angeordnet.

Die Druckschrift EP 2 284 044 A1 beschreibt ein Fahrzeug und Verfahren zum Betreiben eines abgestellten Fahrzeugs mit unbeaufsichtigten Kindern oder Tieren. Es wird in einem parkenden Fahrzeug mittels einer Erfassungseinrichtung in Form einer Kamera und eines Mikrofones mindestens eine Größe in Form eines Videobildes oder einer Tonaufnahme erfasst, welche mit mindestens einem Merkmal des Lebewesens korreliert ist. Die Verbindung mit einer Empfangsstation zur Darstellung des Videobildes und der Tonaufnahme erfolgt mittels WLAN-Verbindung, GSM, UMTS oder einer vergleichbaren Funkverbindung. Das Verfahren eignet sich insbesondere zum Überwachen eines in dem Fahrzeug schlafenden Kindes durch eine Person außerhalb des Fahrzeugs, um zu überprüfen, ob das Kind erwacht ist.

Eine Vorrichtung zur Durchführung von Fahrerzustandsanalysen ist aus der Druckschrift DE 10 2012 002 037 A1 bekannt. Es werden über den Stress-, Gesundheits- und Müdigkeitszustand des Fahrers über den gesamtem Fahrzeitraum, auch über längere Zeiträume, aussagefähige Daten betreffend den Zustand des Fahrers erfasst und über ein Ampelsystem dargestellt.
Die Vorrichtung analysiert und wertet die Herzfrequenzvariabilität aus, wobei die Vitaldaten, insbesondere die EKG-Signal-Daten über textile kapazitive EMG-Sensoren in oder an Ruheelementen des Fahrers angeordnet sind und so eine berührungslose Erfassung der Vitaldaten, insbesondere der EKG-Signal-Daten erfolgt, wobei die Sensoren insbesondere in der Rückenlehne des Fahrzeugsitzes angeordnet sind.

In der Druckschrift DE 10 2016 206 126 A1 wird ein Verfahren und eine Vorrichtung zum Überwachen oder Regeln einer Fahraufgabe-Übergabe in einem selbstfahrenden Fahrzeug und ein System für eine Fahraufgabe-Übergabe in einem selbstfahrenden Fahrzeug beschrieben. Das Verfahren ist für ein selbstfahrendes Fahrzeug vorgesehen. Dabei kann durch das Verfahren die Fahraufgabe-Übergabe überwacht, geregelt oder auch gesteuert werden. Dabei lässt sich das Fahrzeug mithilfe geeigneter im Fahrzeug verbauter Sensoren und Steuergeräte autonom im Verkehrsraum bewegen, wobei eine Fahrzeugführungsaktivität eines Fahrers des Fahrzeugs vollständig unterbleibt. Der Fahrer ist dann für diesen Zeitraum nicht mit der Fahraufgabe betraut und lediglich passiver Passagier im Fahrzeug. Bei der Fahraufgabe-Übernahme-Information und/oder der Sensorinformation handelt es sich um verarbeitete Daten von Signalen von geeignet im Fahrzeug angeordneten Sensoren. Beispielsweise kann die Fahraufgabe-Übernahme-Information unter Beteiligung von Daten von im Lenkrad des Fahrzeugs verbauten Berührungssensoren gebildet werden. Die Sensorinformation kann unter Beteiligung von Daten von im Fahrzeug verbauten optischen Sensoren gebildet werden. Unter einer einem Fahrzeugsitz des Fahrzeugs zugeordneten Schnittstelle kann in diesem Zusammenhang eine Schnittstelle verstanden werden, die eine Information von oder über einen Insassen einliest, der auf dem Fahrzeugsitz sitzt, die der Schnittstelle zugeordnet ist. Als Sensor kann ein 3D-Sensor zur Anwendung kommen, wobei die Sensorinformation über eine Schnittstelle zu dem 3D-Sensor des Fahrzeugs eingelesen werden. Ein 3D-Sensor eignet sich besonders für die schnelle und unaufwändige Klassifizierung von Fahrzeuginsassen.

Aus der Druckschrift DE 10 2005 060 054 B4 ist ein Stereo-Vision-System bekannt, das insbesondere für Insassenrückhaltesysteme mit einem Airbag in Fahrzeugen konzipiert ist. Für die Erfassung von Insassen und deren Sitzposition im Fahrzeug ist eine Vielzahl von Sensoren zur Erzeugung von Bildern vorgesehen. Mit einer Verarbeitungseinheit wird aus den erzeugten Bildern eine Stereoabbildung generiert, welche den räumlichen Abstand der Bildpunkt-Paare enthält. Diese Parameter werden zur Auslösung des Airbags berücksichtigt. Aus der Druckschrift US2007/176402 ist ein optisches Überwachungssystem für eine Fahrgastzelle in einem Auto bekannt, wobei mittels Bildverarbeitung die Position des Fahrers und des Beifahrers ausgewertet wird um u.a. die Auslösung des Airbags zu kontrollieren.

Der Begriff "Stereo Vision" bezeichnet eine Extraktion von 3D-Informationen aus mehreren 2D-Ansichten einer Szene. Die hierfür bekannten konventionellen Stereo-Kameras bestehen üblicherweise aus zwei parallel zueinander ausgerichteten Kameras, so dass ein weitgehend ähnliches Prinzip realisiert wird wie beim menschlichen Stereo-Sehen. Eine angeschlossene Verarbeitungseinheit löst die Bilderfassung der Kameras synchron aus und verarbeitet die erfassten Bilddaten zu 3D-Informationen. Hierfür kommen Algorithmen der Bildauswertung zum Einsatz, welche den visuellen Versatz der Bilder ermitteln.

Aufgabe der Erfindung ist es, eine Vorrichtung und ein Verfahren zur Überwachung einer Fahrgastzelle zu entwickeln, welche einen einfachen konstruktiven Aufbau aufweist und mittels Personenerkennung Notfälle erkennt.

Diese Aufgabe wird mit den kennzeichnenden Merkmalen des ersten und achten Patentanspruchs gelöst.

Vorteilhafte Ausgestaltungen ergeben sich aus den Unteransprüchen.

Die Vorrichtung weist einen optischen Sensor zur Überwachung einer Fahrgastzelle eines Fahrzeuges und deren Insassen auf, wobei der optische Sensor zur Erfassung von Bildinformationen in Form von 2D-Daten und Entfernungsinformationen in Form von 3D-Daten oberhalb der Sitze angeordnet ist. Mit dem Sensor sind die Anzahl der Personen in der Fahrgastzelle und deren Sitzposition und Lage erkennbar. Der Sensor ist mit einer Verarbeitungseinrichtung verbunden oder in Form einer kombinierten Sensor-/Verarbeitungseinrichtung ausgebildet, wobei mittels der Verarbeitungseinrichtung die Bildinformationen in Form von 2D-Daten und Entfernungsinformationen in Form von 3D-Daten in Zustandsinformationen der Personen umwandelbar sind. Die Zustandsinformationen umfassen Informationen in Form von Zustand der Personen und Art und Ort der Positionierung der Personen in der Fahrgastzelle zum Klassifizieren eines Notfalls, wobei der Sensor ein hemisphärisch abbildender 3D-Sensor ist, mit dem die komplette Fahrgastzelle erfassbar ist und wobei der Sensor einen ersten Öffnungswinkel von 135° und einen orthogonal zu dem ersten Öffnungswinkel angeordneten zweiten Öffnungswinkel von 180° aufweist.

Als Zustand kann hierbei die Körperhaltung, Position, Bewegungsmuster oder Vitaldaten verstanden werden.

Mit dem genannten Sensor kann eine Erkennung von Notfällen in Form von Bewegungslosigkeit, Liegen usw., Sitzplatzbelegung und Vitalparameter in Form von beispielsweise Herzschlag von Personen im Fahrzeug erfolgen.

Die Verarbeitung der 2D/3D-Daten und zugehörigen Daten findet vorzugsweise direkt im Gerät statt, was die Notwendigkeit einer Ausgabe von Bildinformationen unnötig macht. Dies dient insbesondere dem Schutz der Privatsphäre der Insassen.

Mittels der Verarbeitungseinrichtung sind die Informationen derart auswertbar, dass mit der Verarbeitungseinrichtung eine Klassifizierung des Zustands der Personen erzeugbar ist und entsprechend der Klassifizierung eine Kommunikation mit einem definierten Empfänger unter Bereitstellung der Erfassungsdaten erfolgt.

Die Kommunikation erfolgt vorzugsweise mittels eines fahrzeuginternen Kommunikationssystems beziehungsweise als Anbindung an das fahrzeuginterne ECall-System oder mittels eines sensorinternen Kommunikationssystems. Die Kommunikation erfolgt in Form von abstrahierten Zustandsdaten derart, dass eine Entscheidung über einen Notfall getroffen wird, die von dem System mit ja/nein eingeordnet wird.

Der Sensor ist in Form eines hemisphärisch abbildenden 3D-Sensors ausgebildet, wobei mittels des 3D-Sensors die komplette Fahrgastzelle erfassbar ist. Die Erfassung der Fahrgastzelle erfolgt mittels omnidirektionaler (= extrem weitwinkeliger) Stereo-Vision basierter 3D-Sensorik. Der Sensor weist hier einen ersten Öffnungswinkel von 135° und einen orthogonal zu dem ersten Öffnungswinkel angeordneten zweiten Öffnungswinkel von 180° auf.

Je nach Anordnung des Sensors ist so der Öffnungswinkel >=135° x 180° (horizontal x vertikal) bzw. >=135° x 180° (vertikal x horizontal).

So können möglichst alle Insassen im Fahrzeuginnenraum sowie ihre Position, ihre Bewegungen und ihr Zustand beziehungsweise ihre Körperhaltung mit einem einzigen Gerät erfasst werden. Bevorzugt funktioniert der Sensor sowohl bei Tageslicht als auch bei Nacht beziehungsweise kompletter Dunkelheit mit aktiver Beleuchtung in Form von IR-Beleuchtung.

Die Bilddaten sind derart umwandelbar, dass die Personen in Form abstrahierter Bewegungs-, Positions- und Körperhaltungsdaten inklusive Gelenkinformationen verarbeitbar sind.

Des Weiteren sind mit dem Sensor Gegenstände von Personen unterscheidbar.

Der optische 3D-Sensor ist bevorzugt derart aufgebaut, dass er aus zwei separaten und miteinander in Wirkverbindung stehenden Kameras gebildet ist, die ein hemisphärisches Umfeld in Echtzeit erfassen und die in einer gemeinsamen Baugruppe baulich integriert sind. In dieser gemeinsamen Baugruppe ist weiterhin eine den zwei Kameras angeschlossene Verarbeitungseinheit baulich integriert, welche die von den zwei Kameras des 3D-Sensors erfassten Bilddaten und 3D-Daten auswertet. Damit wird es möglich, die Fahrgastzelle mit einem einzigen Sensor komplett in drei Dimensionen zu erfassen und auf dem Sensor auszuwerten, das heißt Personen zu erkennen, Objekte zu erkennen und Algorithmen zur Szenenanalyse zu prozessieren.

Das System weist vorzugsweise einen integrierten Prozessor (Embedded-Prozessor/System on Chip) mit CPU und/oder GPU und/oder ASIC und/oder FPGA und/oder ARM-Prozessor auf, der für Berechnungen unter Nutzung eines Stereokorrespondenzverfahrens, zur Berechnung von Tiefen- und 3D-Informationen (x-y-z) und für kundenspezifische Analysesoftware und/oder Applikationen mit Auswertung der Tiefen- und/oder 3Dlnformationen, mit applikationsspezifischer Datenauswertung und mit Informationsgenerierung geeignet ist.

Bei dem erfindungsgemäßen Verfahren erfolgt die Überwachung einer Fahrgastzelle eines Fahrzeuges und deren Insassen mittels eines optischen Sensors, wobei der Sensor oberhalb der Insassen angeordnet ist. Der Sensor weist eine Personenerkennung auf und erfasst Bildinformationen in Form von 2D-Daten und Entfernungsinformationen in Form von 3D-Daten zu den Insassen. Über eine Verarbeitungseinrichtung werden die erfassten Daten in Informationen über den Zustand und/oder die Position und/oder die Haltung der Insassen im Fahrzeug umgewandelt, wobei anhand der Informationen eine Klassifizierung von Notfällen erfolgt.

Im Falle eines Notfalls erfolgt eine Kommunikation mit Übermittlung der Zustandsdaten mit einem externen Empfänger in Form einer Notfallzentrale oder einer hinterlegten Nummer.

Die Kommunikation erfolgt mittels eines fahrzeuginternen oder sensorinternen Kommunikationssystems. Bei der Nutzung fahrzeuginterner Kommunikationssysteme kann insbesondere das fahrzeuginterne ECall-System genutzt werden. Die Kommunikation erfolgt mittels abstrahierter Zustandsdaten, sodass eine Beurteilung erfolgen kann, Notfall ja/nein.
Ein Sensorinternes Kommunikationssystem kann kabelgebunden oder drahtlos implementiert sein. Für eine kabelgebundene Ausführung eignet sich insbesondere Ethernet (802.3), KNX, CAN, UART/RS232 oder USB. Auch eine Kombination der Systeme ist möglich. Die drahtlose Übertragung erfolgt mittels WLAN (802.11), Bluetooth, Zigbee, Z-WAVE, GSM oder UMTS.

Die Überwachung mittels des Sensors ist derart mit dem Fahrzeug kombiniert, dass in Abhängigkeit der Informationen des Sensors das Fahrzeug selbstständig anhalten kann. Dies ist insbesondere bei autonomen Fahrzeugen möglich. Des Weiteren ist das Anhalten des Fahrzeuges möglich, sobald ein Insasse versucht, eine Tür zu öffnen.

Die Erkennung der Personen und ihrer Haltungen erfolgt insbesondere mit Hilfe KI-basierter Algorithmen aus den Bild- und Entfernungsinformationen. Der genutzte Sensor ist hier bevorzugt ein hemisphärisch abbildender 3D-Sensor unter Nutzung von Stereo-Vision, sodass die Überwachung der Fahrgastzelle mittels eines einigen Sensors erfolgen kann.

Die erfindungsgemäße Lösung gewährleistet die Überwachung des Zustandes von Insassen im Fahrzeug mit der optischen Sensorik und ermöglicht die Erkennung von Notfällen (Bewegungslosigkeit, Liegen usw.), Sitzplatzbelegung und perspektivisch bspw. Vitalparameter (bspw. Herzschlag) von Personen im Fahrzeug.
Vorteilhafter Weise kann mit nur einem Sensor die Überwachung der kompletten Fahrgastzelle eines PKWs mit zwei Sitzreihen durch Nutzung Hemisphärischer Stereo-Vision Sensortechnologie realisiert werden. Bei weiteren Sitzreihen des PKWs oder bei der Überwachung beispielsweise von Kleinbussen oder auch anderen Bussen, Straßenbahnen oder anderen Schiebenfahrzeugen können, wenn nötig, mehrere Sensoren kombiniert werden.

Eine derartige Vorrichtung und Verfahren eignen sich insbesondere für das autonome Fahren, wobei erkennbar ist, wie viele Personen und in welchem Zustand die Personen im Fahrzeug sind. Die Vorrichtung kann auch zur Unfallvermeidung eingesetzt werden, beispielsweise zum Stoppen des Fahrzeuges, wenn sich andere Fahrzeuge auf die Türen zubewegen.

Des Weiteren eignen sich die Vorrichtung und das Verfahren für Sonderfahrzeuge derart, dass nach Beschuss oder Unfall des Fahrzeuges der Zustand der Personen im Fahrzeug erkennbar und übermittelbar ist.

Die Erfindung wird nachfolgend an einem Ausführungsbeispiel und zugehörigen Zeichnungen näher erläutert.

Es zeigen:
- Figur 1: eine beispielhafte Anordnung eines hemisphärisch abbildenden 3D-Sensors in einem PKW,
- Figur 2: eine Darstellung der abstrahierten Bewegungs-, Positions- und Körperhaltungsdaten inklusive Gelenkinformationen in einer Bildinformation,
- Figur 3: ein Schema des Systems zur Verarbeitung der mit dem Sensor erfassten Daten.

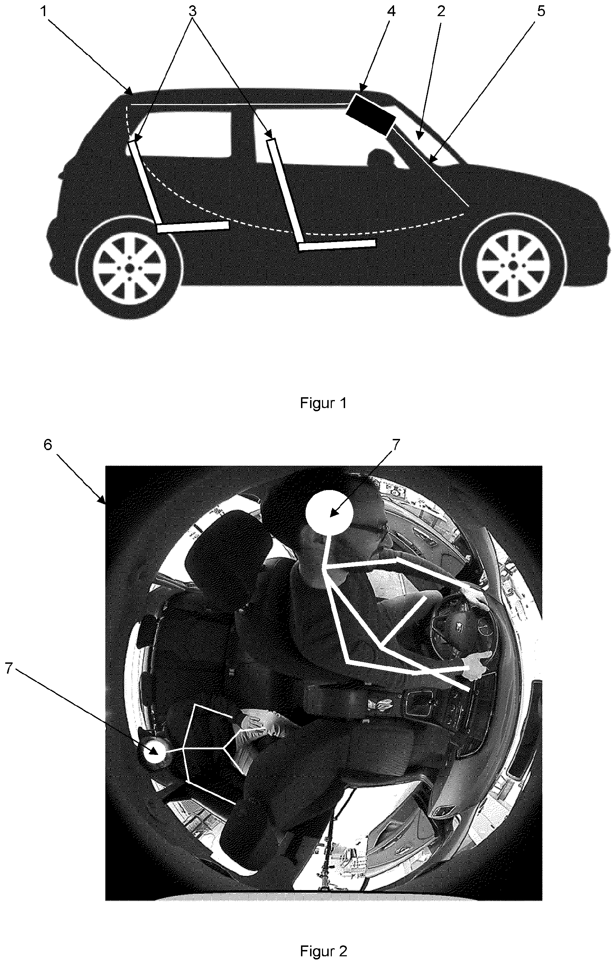

Gemäß Figur 1 wird die Fahrgastzelle des dargestellten PKWs bekannter Weise von einem Dach 1, einer Windschutzscheibe 2, einem Armaturenbrett und einer Fußablage begrenzt. Weiterhin sind Fahrzeugsitze 3 in einer ersten und zweiten Sitzreihe vorgesehen, auf denen Person beziehungsweise die Insassen sitzen können. Im vorderen Bereich des Daches 1 und/oder im oberen Bereich der Windschutzscheibe 2 bzw. im mittleren vorderen Bereich im Dachhimmel ist der hemisphärisch abbildende 3D-Sensor 4 angeordnet, der die gesamte Fahrgastzelle erfasst. Der mittels des Sensors erfassbare Bereich 5 ist schematisch in der Figur 1 dargestellt und erstreckt sich entlang der Frontscheibe 2 und des Dachhimmels und bildet einen Teilkreis im Innenraum, der bis an die Sitze 5 oder darüber hinaus in Richtung der Fußablagen reicht.

Die Auswertung der Daten erfolgt im Sensor 4 selbst, so dass der Sensor 4 keine Bilddaten der im Fahrzeug befindlichen Personen und/oder Gegenstände ausgibt. Mit dem Sensor 4 wird die Erkennung von Personen und ihrer Haltungen mit Hilfe Kl-basierter Algorithmen aus Bild- und 3D-Informationen realisiert. So kann eine Bewegungs- und Körperhaltungsschätzung sowie Vitalparameterbestimmung erfolgen.
Dadurch ist es möglich, eine Zustandsmessung von Fahrzeuginsassen mit Bezug zum 3D-Raum des Fahrzeuges beziehungsweise in der Fahrgastzelle zu realisieren und auf Basis der Auswertung dieser Daten Notfälle zu klassifizieren.

In Figur 2 ist eine Darstellung einer Bildinformation 6 beziehungsweise ein Foto dargestellt, welches mittels des optischen Sensors erfasst wird wobei im Folgenden die Bilddaten umgewandelt werden, sodass die Personen in Form abstrahierter Bewegungs-, Positions- und Körperhaltungsdaten inklusive Gelenkinformationen darstellbar sind. Dies entspricht gemäß Figur 2 einer Strichdarstellung 7.

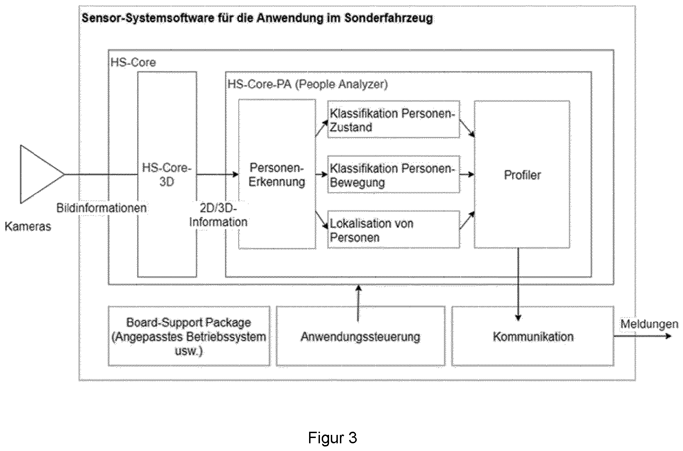

In Figur 3 ist der schematische Ablauf zur Verarbeitung der mit dem Sensor erfassten Daten dargestellt. Mittels wenigstens zweier Kameras werden Bildinformationen aufgenommen und folgend mit Hilfe einer CPU (HS-Core-3D) in Bilddaten und Entfernungsdaten beziehungsweise 2D- und 3D-Informationen umgewandelt. Die Bilddaten beziehungsweise Bildinformationen werden derart umgewandelt, dass die Personen in Form abstrahierter Bewegungs-, Positions- und Körperhaltungsdaten inklusive Gelenkinformationen verarbeitet werden, sodass keine Bildinformationen über die Insassen gespeichert werden, wodurch Schutz der Privatsphäre der Insassen gewährleistet ist.

Mit den ermittelten 2D- und 3D-Informationen erfolgt in der CPU eine Personenerkennung und die Beurteilung der Person mittels dreier Kriterien. Diese Kriterien umfassen die Klassifikation nach dem Zustand der Person, wobei insbesondere Vitalzeichen überprüft werden.

Als zweites Kriterium erfolgt die Klassifikation nach der Bewegung, das heißt ob die Person sich noch bewegt oder beispielsweise ohnmächtig ist. Das dritte Kriterium ist die Lokalisierung der Person, das heißt ob die Person liegt oder sitzt.
Mit den ermittelten Daten wird mittels eines Profilers eine Entscheidung über den Zustand der Personen getroffen, wobei bei der Entscheidung eines Notfalls eine Kommunikation erfolgt derart, dass eine Meldung beziehungsweise ein Notruf abgesetzt wird.

Die Verarbeitung in der Verarbeitungseinrichtung erfolgt unter Zuhilfenahme eines angepassten Betriebssystems (Board-Support Package) und mit einer Anwendungssteuerung, die je nach benötigter Anwendung programmierbar ist.

### Bezugszeichenliste

- 1: Dach
- 2: Windschutzscheibe
- 3: Fahrzeugsitz
- 4: 3D-Sensor
- 5: Erfassbarer Bereich
- 6: Bildinformation
- 7: Strichdarstellung der Personen

## Patentansprüche

1. Vorrichtung zur Überwachung einer Fahrgastzelle eines Fahrzeuges (5) und deren Insassen, wobei ein optischer Sensor (4) zur Erfassung von Bildinformationen in Form von 2D-Daten und Entfernungsinformationen in Form von 3D-Daten oberhalb der Sitze angeordnet ist und dass mit dem Sensor die Anzahl der Personen in der Fahrgastzelle und deren Sitzposition erkennbar sind und dass der Sensor mit einer Verarbeitungseinrichtung verbunden oder eine Verarbeitungseinrichtung in dem Sensor angeordnet ist, wobei mittels der Verarbeitungseinrichtung die 2D- und 3D-Daten in Zustandsinformationen der Personen umwandelbar sind und dass die Zustandsinformationen Informationen in Form von Zustand der Personen und Art und Ort der Positionierung der Personen in der Fahrgastzelle zum Klassifizieren eines Notfalls umfassen und wobei der Sensor ein hemisphärisch abbildender 3D-Sensor ist, mit dem die komplette Fahrgastzelle erfassbar ist und wobei der Sensor einen ersten Öffnungswinkel von 135° und einen orthogonal zu dem ersten Öffnungswinkel angeordneten zweiten Öffnungswinkel von 180° aufweist.

2. Vorrichtung nach Anspruch 1, **dadurch gekennzeichnet, dass** mittels der Verarbeitungseinrichtung die Informationen auswertbar sind derart, dass mit der Verarbeitungseinrichtung eine Klassifizierung des Zustands der Personen erzeugbar ist und entsprechend der Klassifizierung eine Kommunikation mit einem definierten Empfänger unter Bereitstellung von Erfassungsdaten erzeugbar ist.

3. Vorrichtung nach Anspruch 2, **dadurch gekennzeichnet, dass** die Kommunikation mittels eines fahrzeuginternen Kommunikationssystems oder mittels eines sensorinternen Kommunikationssystems erfolgt.

4. Vorrichtung nach einem der Ansprüche 1 bis 3, **dadurch gekennzeichnet, dass** die Bilddaten derart umwandelbar sind, dass die Personen in Form abstrahierter Bewegungs-, Positions- und Körperhaltungsdaten inklusive Gelenkinformationen verarbeitbar sind.

5. Vorrichtung nach einem der Ansprüche 1 bis 4, **dadurch gekennzeichnet, dass** mit dem Sensor Gegenstände von Personen unterscheidbar sind.

6. Verfahren zur Überwachung einer Fahrgastzelle eines Fahrzeuges und deren Insassen mittels eines optischen Sensors, wobei der Sensor oberhalb der Insassen angeordnet ist, wobei der Sensor eine Personenerkennung aufweist und Bildinformationen in Form von 2D-Daten und Entfernungsinformationen in Form von 3D-Daten zu den Insassen erfasst und dass über eine Verarbeitungseinrichtung die erfassten Daten in Informationen über den Zustand und/oder die Position und/oder die Haltung der Insassen im Fahrzeug umgewandelt werden und dass anhand der Informationen eine Klassifizierung von Notfällen erfolgt, und wobei die Überwachung der kompletten Fahrgastzelle mittels eines einzigen hemisphärisch abbildenden 3D-Sensors unter Nutzung von Stereo-Vision erfolgt.

7. Verfahren nach Anspruch 6, **dadurch gekennzeichnet, dass** im Falle eines Notfalls eine Kommunikation mit Übermittlung der Zustandsdaten mit einem externen Empfänger in Form einer Notfallzentrale oder einer hinterlegten Nummer erfolgt.

8. Verfahren nach Anspruch 7, **dadurch gekennzeichnet, dass** die Kommunikation mittels eines fahrzeuginternen oder sensorinternen Kommunikationssystems erfolgt.

9. Verfahren nach einem der Ansprüche 6 bis 8, **dadurch gekennzeichnet, dass** die Überwachung mit dem Fahrzeug derart kombiniert ist, dass in Abhängigkeit der Informationen des Sensors das Fahrzeug selbstständig anhalten kann.

10. Verfahren nach einem der Ansprüche 6 bis 9, **dadurch gekennzeichnet, dass** die Erkennung der Personen und ihrer Haltungen mit Hilfe Kl-basierter Algorithmen aus den Bild- und Entfernungsinformationen erfolgt.

## Claims

1. Device for monitoring a passenger compartment of a vehicle and its occupants, wherein an optical sensor (4) for detecting image information in the form of 2D data and distance information in the form of 3D data is arranged above the seats, and in that the number of persons in the passenger compartment and their seat position can be detected by means of the sensor, and in that the sensor is connected to a processing device or a processing device is arranged in the sensor, wherein the 2D and 3D data can be converted into status information of the persons by means of the processing device, and in that the status information comprises information in the form of the status of the persons and the type and location of the positioning of the persons in the passenger compartment for classifying an emergency, and wherein the sensor is a hemispherically imaging 3D sensor with which the complete passenger compartment can be detected, and wherein the sensor has a first aperture angle of 135° and a second aperture angle of 180° arranged orthogonally to the first aperture angle.

2. Device according to claim 1, **characterized in that** the information can be evaluated by means of the processing device in such a way that the processing device can be used to generate a classification of the status of the persons and, in accordance with the classification, a communication with a defined receiver can be generated with the provision of detection data.

3. Device according to claim 2, **characterized in that** the communication takes place by means of an in-vehicle communication system or by means of a sensor-internal communication system.

4. Device according to one of claims 1 to 3, **characterized in that** the image data can be converted in such a way that the persons can be processed in the form of abstracted movement, position and posture data including joint information.

5. Device according to one of claims 1 to 4, **characterized in that** objects can be distinguished from persons by means of the sensor.

6. Method for monitoring a passenger compartment of a vehicle and its occupants by means of an optical sensor, wherein the sensor is arranged above the occupants, wherein the sensor has a person recognition system and acquires image information in the form of 2D data and distance information in the form of 3D data relating to the occupants, and in that the acquired data are converted via a processing device into information about the status and/or the position and/or the posture of the occupants in the vehicle, and in that a classification of emergencies is carried out on the basis of the information, and wherein the monitoring of the complete passenger compartment is carried out by means of a single hemispherically imaging 3D sensor using stereo vision.

7. Method according to claim 6, **characterized in that**, in the event of an emergency, communication with transmission of the status data takes place with an external receiver in the form of an emergency center or a stored number.

8. Method according to claim 7, **characterized in that** the communication takes place by means of an in-vehicle or sensor-internal communication system.

9. Method according to one of claims 6 to 8, **characterized in that** the monitoring is combined with the vehicle in such a way that, depending on the information from the sensor, the vehicle can stop independently.

10. Method according to one of claims 6 to 9, **characterized in that** the recognition of the persons and their postures is performed with the aid of AI-based algorithms from the image and distance information.

## Revendications

1. Dispositif pour la surveillance de l'habitacle d'un véhicule (5) et de ses occupants, dans lequel un capteur optique (4) destiné à acquérir des informations d'image sous forme de données 2D et des informations de distance sous forme de données 3D est disposé au-dessus des sièges et dans lequel le capteur peut détecter le nombre de personnes dans l'habitacle et leur emplacement de siège et dans lequel le capteur communique avec une installation de traitement ou bien une installation de traitement est disposée dans le capteur, les données 2D et 3D pouvant être converties en informations sur l'état des personnes au moyen de l'installation de traitement, et dans lequel les informations d'état sont des informations sur l'état des personnes et sur le mode et le lieu de positionnement des personnes dans l'habitacle pour la classification d'une situation d'urgence, le capteur étant un capteur 3D à champ hémisphérique qui peut acquérir l'habitacle complet et le capteur présentant un premier angle d'ouverture de 135° et un deuxième angle d'ouverture de 180° disposé perpendiculairement au premier angle d'ouverture.

2. Dispositif selon la revendication 1, **caractérisé en ce que** les informations peuvent être analysées au moyen de l'installation de traitement de telle manière que l'installation de traitement puisse générer une classification de l'état des personnes et puisse générer en fonction de la classification une communication avec un récepteur défini en fournissant des données d'acquisition.

3. Dispositif selon la revendication 2, **caractérisé en ce que** la communication a lieu au moyen d'un système de communication interne au véhicule ou au moyen d'un système de communication interne au capteur.

4. Dispositif selon l'une des revendications 1 à 3, **caractérisé en ce que** les données d'images peuvent être converties de telle façon que les personnes peuvent être traitées sous la forme de données abstraites de mouvement, de position et de posture corporelle incluant des informations sur leurs articulations.

5. Dispositif selon l'une des revendications 1 à 4, **caractérisé en ce que** le capteur peut différencier les objets des personnes.

6. Procédé pour la surveillance de l'habitacle d'un véhicule et de ses occupants au moyen d'un capteur optique, dans lequel le capteur est disposé au-dessus des occupants, le capteur présentant une reconnaissance des personnes et acquérant des informations d'image sous forme de données 2D et des informations de distance sous forme de données 3D, dans lequel une installation de traitement convertit les données acquises en informations sur l'état et/ou la position et/ou la posture des occupants du véhicule et dans lequel une classification des situations d'urgence est effectuée à l'aide des informations, la surveillance de l'habitacle entier étant réalisée au moyen d'un seul capteur 3D à champ hémisphérique utilisant une vision stéréoscopique.

7. Procédé selon la revendication 6, **caractérisé en ce qu'**en cas d'urgence, une communication avec transmission des données d'état est établie avec un récepteur externe qui peut être un centre d'appel d'urgence ou un numéro enregistré.

8. Procédé selon la revendication 7, **caractérisé en ce que** la communication a lieu au moyen d'un système de communication interne au véhicule ou interne au capteur.

9. Procédé selon l'une des revendications 6 à 8, **caractérisé en ce que** la surveillance est combinée avec le véhicule de telle manière que le véhicule puisse s'arrêter de lui-même en fonction des informations du capteur.

10. Procédé selon l'une des revendications 6 à 9, **caractérisé en ce que** la reconnaissance des personnes et de leur posture est réalisée à l'aide d'algorithmes KI à partir des informations d'images et de distance.
